Europäisches Patentamt

European Patent Office

Office européen des brevets

(19)

(11) Veröffentlichungsnummer: **0 173 951 B1**

## EUROPÄISCHE PATENTSCHRIFT

(12)

(45) Veröffentlichungstag der Patentschrift: **19.06.91**

(51) Int. Cl.⁵: **A61K 37/54, A61K 39/385**

(21) Anmeldenummer: **85110757.3**

(22) Anmeldetag: **27.08.85**

(54) Tumortherapeutikum und Verfahren zu seiner Herstellung.

(30) Priorität: **06.09.84 DE 3432714**

(43) Veröffentlichungstag der Anmeldung:
**12.03.86 Patentblatt 86/11**

(45) Bekanntmachung des Hinweises auf die
Patenterteilung:
**19.06.91 Patentblatt 91/25**

(84) Benannte Vertragsstaaten:
**AT BE CH DE FR GB IT LI LU NL SE**

(56) Entgegenhaltungen:
EP-A- 0 019 167   EP-A- 0 141 079
EP-A- 0 160 897   WO-A-82/04263
DE-A- 2 643 215   FR-A- 2 323 147
GB-A- 1 592 496   US-A- 4 446 122

BIOLOGICAL ABSTRACTS, Band 57, Nr. 6,
1984, Seite 34, Nr. 32492, Biological Abstracts Inc., Philadelphia, US; G.A. CURRIE:
"Effect of active immunization with irradiated tumour cells on specific inhibitors of
cell-mediated immunity in patients with disseminated cancer"

NATURE, Band 248, 19. April 1974, Seiten
690-691; C.J. SANDERSON et al.: "The induction of tumour immunity in mice using

glutaraldehyde-treated tumour cells"

(73) Patentinhaber: **BEHRINGWERKE Aktiengesellschaft
Postfach 1140
W-3550 Marburg 1(DE)**

(72) Erfinder: **Bosslet, Klaus, Dr.
Ockershäuser Allee 5
W-3550 Marburg 1(DE)**
Erfinder: **Sedlacek, Hans-Harald, Dr.
Am Sonnenhang 3
W-3550 Marburg 1(DE)**

(74) Vertreter: **Becker, Heinrich Karl Engelbert, Dr.
et al
HOECHST AKTIENGESELLSCHAFT Central
Patent Department P.O. Box 80 03 20
W-6230 Frankfurt am Main 80(DE)**

Rank Xerox (UK) Business Services

**Beschreibung**

Die Erfindung betrifft ein Verfahren zur Herstellung eines Therapeutikums aus Tumorzellen zur Therapie von Tumorerkrankungen sowie ein solches Therapeutikum.

Es ist beispielsweise aus "Mechanisms of Tumor Immunity, Gree et al., eds., John Wiley Sons, N.Y., 1977, Seite 196, bekannt, daß bereits versucht wurde, Tumorerkrankungen durch Impfen mit Tumorzellen, die durch Gefrieren und Auftauen, Lyophilisieren, Druck oder Homogenisieren modifiziert waren, zu therapieren. Auch subzelluläre Fraktionen oder Zellextrakte wurden zu diesem Zweck verwendet. Aus Nature, Bd. 248 (1974), 690-691, ist auch die Behandlung von durch Methylcholanthren (Meth A) induzierten transplantierbaren Maus-Tumoren (Meth A Tumoren) mit Glutardialdehyd bekannt.

Es ist jedoch bisher kein Impfstoff gegen eine Tumorerkrankung bekannt.

Wir haben überraschenderweise gefunden, daß lyophilisierte oder mit einem Aldehyd behandelte Zellen aus menschlichen Tumoren oder aus solchen gewonnene Zellaggregate, die Antigene CEA oder NCA tragen, als Therapeutikum zur Behandlung von Tumorerkrankungen benutzt werden können.

Gegenstand der Erfindung ist deshalb ein Therapeutikum zur Behandlung einer Tumorerkrankung, enthaltend getrocknete oder durch eine chemische Behandlung stabilisierte menschliche Zellen, die Antigene CEA oder NCA tragen.

Ein Vorteil eines solchen Therapeutikums gegenüber einer Verwendung von nativen Zellen besteht darin, daß native Zellen nicht stabil sind, so daß sie jeweils frisch hergestellt werden müssen. Sie können deshalb auch nicht standardisiert werden.

Gegenstand der Erfindung ist weiterhin ein Verfahren zur Herstellung eines Therapeutikums zur Behandlung einer Tumorerkrankung, dadurch gekennzeichnet, daß menschliche Tumorzellen, die Antigene CEA oder NCA tragen, getrocknet oder durch eine chemische Behandlung stabilisiert und zu einem Therapeutikum aufgearbeitet werden, wobei gegebenenfalls Neuraminidase zugegeben wird.

Die Möglichkeit, eine Immunantwort durch Neuraminidase zu verstärken, ist aus der deutschen Offenlegungsschrift 26 20 649 bekannt.

Als Therapeutikum ist ein Mittel zu verstehen, das sowohl prophylaktisch als auch zur Behandlung einer manifesten Erkrankung geeignet sein kann.

Die im Rahmen der Erfindung verwendbaren Zellen werden nach bekannten Zellkulturverfahren aus Tumoren gewonnen. Auf mechanische oder enzymatische Weise aus solchen Kulturen gewonnene, gegebenenfalls mit Mitomycin C inaktivierte Zellen werden getrocknet, vorzugsweise lyophilisiert, oder mit einem dem Fachmann als Stabilisierungsmittel für organische Gewebe bekannten Agenz, vorzugsweise einem Mono- oder Dialdehyd mit 1 bis 6 Kohlenstoffatomen, behandelt.

Zu den zur chemischen Stabilisierung und Fixierung besonders geeigneten Agenzien gehören besonders bifunktionelle Verbindungen, also solche, die zwei Gruppen enthalten, die mit funktionellen Gruppen auf dem biologischen Material reagieren - es also "vernetzen" - können. Das sind beispielsweise Dialdehyde, besonders aliphatische Dialdehyde mit 2-8 Kohlenstoffatomen. Dazu sind jedoch auch geeignet Monoalkanale mit 1-4 Kohlenstoffatomen, wie Formaldehyd, der bifunktionell reagieren kann, aber auch bifunktionelle Immunoester wie Suberomidat, Isocyanate oder Isothiocyanate.

Als Agenzien, die biologisches Material stabilisieren können, können auch sogenannte Gerbmittel dienen wie zum Beispiel Gerbsäure und ihre Derivate oder Chromsalze. Auch Sulfosalicylsäure ist geeignet.

Im allgemeinen liegen die Zellen als Zellklumpen oder als Einzelzellen vor.

Beispiele für die Antigene sind CEA (carcinoembryonic antigen; J.exp.Med. (1965) 122, 467) oder NCA (non-specific crossreacting antigen; J.Immun. (1973) III, 1926), welche mittels Immunaffinitätschromatographie aus den Tumorzellen isoliert werden können. Hierzu werden die in der Tabelle I in der deutschen Offenlegungsschrift 34 16 774 beschriebenen monoklonalen Antikörper als gereinigte Proteine kovalent an CNBr-aktivierte Sepharose 4B gebunden und die von diesen monoklonalen Antikörpern erkannten Antigene (CEA, NCA) aus DE-TA Colonkarzinomzellextrakten isoliert. in geeignetes Verfahren ist im Pharmacia Buch "Affinity Chromatography, Principles and Methods", 12-18 (1979), zusammengefaßt auf Seite 15, beschrieben.

Die Qualitätskontrolle eines als Impfstoff zu benutzenden Materials wird beispielsweise durch Typisierung mit monoklonalen Antikörpern, oder durch Auftrennung der Gesamtzellproteine mittels SDS-Polyacrylamidgelelektrophorese oder isoelektrischer Fokussierung (1. Dimension) kombiniert mit SDS-Polyacrylamidgelelektrophorese (2. Dimension) mit nachfolgender Anfärbung des Gels (Silberfärbung) durchgeführt.

Das antigene Material wird bevorzugt in Kombination mit einem Adjuvans, besonders Neuraminidase (Deutsche Offenlegungsschrift 26 20 649), intradermal vorzugsweise nach dem Verfahren der Schachbrettvakzination (Cancer Immunol. and Immunother. 6, 47-58 (1979), spez. S.48) appliziert.

Ein solcher Impfstoff wird vorzugsweise gegen bestimmte Stadien des Colonkarzinoms (Duke C) sowie gegen andere Tumoren eingesetzt, die Antigene oder epitope tragen, welche im Impfstoff vorhanden sind. Solche anderen Tumoren sind solide Tumoren, beispielsweise Pankreaskarzinom, Magenkarzinome, Mammakarzinome und Lungenkarzinome. Der Impfstoff kann parenteral oder oral verabreicht werden. Die Antigene können in physiologischer Kochsalzlösung gelöst oder suspendiert appliziert werden, vorzugsweise intradermal in PBS.

Als Qualitätskriterien für die Stabilität der Antigenzusammensetzung des Impfstoffs wurden zwei Tests durchgeführt:

a) Der Terasaki-IIF-Assay (indirekte Immunfluoreszenz unter Verwendung von Tumorzellen, die in den Näpfchen der Terasaki-Mikrotiterplatte wachsen) mit monoklonalen Antikörpern verschiedener Spezifität. Mittels dieses Tests ist es möglich, die Expression von Membranantigenen auf intakten Tumorzellen zu messen, gegen die eine Reihe monoklonaler Antikörper verfügbar sind (Cancer Detection and Prevention 6, 181-184, 1983). Hiermit ließen sich drastische Veränderungen auf der DE-TA-Zellmembran im Laufe der Kultivierung feststellen; b) Solubilisierung der Gesamtzellproteine durch ein Detergens (Hybridoma 1, 413-421, 1982) sowie nachfolgende SDS-Polyacrylamidgelelektrophorese kombiniert mit Silberfärbung (Anal. Biochem. 105, 361-363, 1980). Die Kombination dieser Techniken stellt sicher, daß keine signifikanten Veränderungen im Gesamtproteingehalt der DE-TA-Zellinie eingetreten sind.

Die folgenden Beispiele erläutern die Erfindung.

Beispiel 1

Die Karzinomzellinie BW X wurde in Zellkultur als Monolayer wachsend in RPMI-1640-Medium (Moore, G.E., Gerner, R.E., Franklin, H.A., Culture of normal human leucocytes, J.A.M.A. 199, 519-524 (1967)) mit 10% foetalem Kälberserum in Plastikflaschen kultiviert. Die adhärent wachsenden Zellen konfluenter Kulturen wurden mechanisch oder mittels Trypsin, das in foetalem Kälberserumfreien RPMI-1640-Medium gelöst war, abgelöst, die Kollagenase durch Zugabe von in RPMI-1640 gelöstem foetalen Kälberserum inaktiviert, anschließend die Zellen aus den Gewebekulturflaschen herausgelöst und 3 mal in 37°C warmer phosphatgepufferter Kochsalzlösung (PBS) gewaschen.

Etwa $10^7$ Zellen, die größerenteils als Klumpen vorliegen, wurden 1 Stunde bei 37°C in 1 ml PBS, das 100 $\mu$g Mitomycin C enthält, inkubiert. Anschließend wurden die so inaktivierten Zellen 3 mal mit PBS gewaschen und

a) 3 mal in 0,18 molarem Ammoniumbicarbonat-Puffer, der mit Essigsäure auf pH 7,4 eingestellt worden war, gewaschen. Ein $10^7$ Zellen entsprechendes Zellsediment wurde in 100 $\mu$l desselben Ammoniumbicarbonat-Puffers, aufgenommen und bei -70°C gefroren. Anschließend wurde das gefrorene Material gefriergetrocknet und bei +4°C im Kühlschrank in einem luftdicht verschlossenen Glasfläschchen gelagert. Das so behandelte Zellmaterial kann, in PBS aufgenommen, in eine Vakzinalion am Patienten eingesetzt werden.

Alternativ wurde

b) 5 Minuten bei +4°C mit 0,1% Glutaraldehyd in PBS inkubiert, der überschüssige Glutaraldehyd durch 3 maliges Waschen mit PBS entfernt, anschließend mit 2% BSA (Bovine Serum Albumin) für 5 Minuten bei +4°C inkubiert und 3 mal in PBS gewaschen. Die so behandelten Zellen können bei +4°C gelagert werden und in die Vakzination am Patienten eingesetzt werden.

Oder es wurde

c) bei 25°C in Formalin nach Lilly (Benno Romeis (1968), S. 65, Kap. 266, Oldenburg Verlag München) unter gelegentlichem Schütteln über Nacht inkubiert. Die Zellen (ca. $10^8$) wurden zentrifugiert, der Überstand dekantiert (10 Minuten bei 800 g) und das Zellsediment in 7 ml doppelt destilliertem Wasser suspendiert (= 1-mal Waschen). Dieser Waschvorgang wurde in 1-stündigen Abständen 4-mal wiederholt. Anschließend wurde das Zellsediment 3-mal in 1-stündigen Abständen in je 7 ml 70%-igem Ethanol gewaschen. Anschließend wurde das Zellsediment 3-mal in 30-minütigen Abständen in je 7 ml 80%igem Ethanol gewaschen. Anschließend wurde das Zellsediment 3-mal in 30-minütigen Abständen in je 7 ml 96%igem Ethanol gewaschen. Anschließend wurde das Zellsediment 3-mal in 30-minütigen Abständen in je 7 ml 99%igem Ethanol gewaschen. Anschließend wurde das Zellsediment 3-mal in 30-minütigen Abständen in je 7 ml sterilem PBS gewaschen und steril bei 4°C aufbewahrt. Die so behandelten Zellen können bei 4°C gelagert werden und in die Vakzination am Patienten eingesetzt werden.

Ansprüche

1. Therapeutikum zur Behandlung einer Tumorerkrankung, enthaltend gefriergetrocknete oder durch eine chemische Behandlung stabilisierte menschliche Tumorzellen, welche die membran-assoziierten Antigene CEA oder NCA tragen.

2. Therapeutikum nach Anspruch 1, dadurch gekennzeichnet, daß es ein die Immunantwort verstärkendes Adjuvans, vorzugsweise Neuraminidase, enthält.

3. Verfahren zur Herstellung eines Therapeutikums zur Behandlung einer Tumorerkrankung, dadurch gekennzeichnet, daß menschliche Tumorzellen, welche die membran-assoziierten Antigene CEA oder NCA tragen, gefriergetrocknet oder durch eine chemische Behandlung stabilisiert und zu einem Therapeutikum aufgearbeitet werden.

4. Verfahren nach Anspruch 3, dadurch gekennzeichnet, daß das Therapeutikum zusätzlich mit einem die Immunantwort verstärkenden Adjuvans, vorzugsweise Neuraminidase, versetzt wird.

5. Verwendung von menschlichen Tumorzellen, welche die membranassoziierten Antigene CEA oder NCA tragen, gegebenenfalls in Kombination mit einem die Immunantwort verstärkenden Adjuvans, vorzugsweise Neuraminidase, zur Herstellung eines Therapeutikums zur Behandlung von Tumorerkrankungen.

## Claims

1. A therapeutic agent for the treatment of a tumorous disease, containing human tumor cells which have been freeze-dried or stabilized by a chemical treatment and which carry the membrane-associated antigens CEA or NCA.

2. A therapeutic agent as claimed in claim 1, which contains an adjuvant which potentiates the immune response, preferably neuraminidase.

3. A process for the preparation of a therapeutic agent for the treatment of a tumorous disease, which comprises the freeze-drying or stabilization by a chemical treatment, and, the processing to a therapeutic agent, of human tumor cells which carry the membrane-associated antigens CEA or NCA.

4. The process as claimed in claim 3, wherein an adjuvant which potentiates the immune response, preferably neuraminidase, is additionally added to the therapeutic agent.

5. The use of human tumor cells which carry the membrane-associated antigens CEA or NCA, where appropriate in combination with an adjuvant which potentiates the immune response, preferably neuraminidase, for the preparation of a therapeutic agent for the treatment of tumorous diseases.

## Revendications

1. Agent thérapeutique pour le traitement d'une maladie tumorale, contenant des cellules tumorales humaines, lyophilisées ou stabilisées par un traitement chimique, qui portent les antigènes CEA ou NCA associés à la membrane.

2. Agent thérapeutique selon la revendication 1, caractérisé en ce qu'il contient un adjuvant accentuant la réponse immunitaire, de préférence de la neuraminidase.

3. Procédé pour la préparation d'un agent thérapeutique pour le traitement d'une maladie tumorale, caractérisé en ce que des cellules tumorales humaines, qui portent les antigènes CEA ou NCA associés à la membrane, sont lyophilisées ou stabilisées par un traitement chimique, puis soumises à un traitement final pour l'obtention d'un agent thérapeutique.

4. Procédé selon la revendication 3, caractérisé en ce que l'agent thérapeutique est en outre additionné d'un adjuvant accentuant la réponse immunitaire, de préférence la neuraminidase.

5. Utilisation de cellules tumorales humaines, qui portent les antigènes CEA ou NCA associés à la membrane, éventuellement en association avec un adjuvant accentuant la réponse immunitaire, de préférence la neuraminidase, pour la préparation d'un agent thérapeutique pour le traitement de maladies tumorales.